# EUROPEAN PATENT SPECIFICATION

(11) **EP 2 731 661 B1**
(45) Date of publication and mention of the grant of the patent: **18.09.2019**
(21) Application number: 12737960.0
(22) Date of filing: 02.07.2012
(51) Int. Cl.: A61M 25/00, A61L 31/06, A61F 2/06, A61F 2/00, A61F 2/958, A61F 2/82, A61M 25/10, A61L 31/16

(54) **DRUG ELUTION MEDICAL DEVICE**
MEDIKAMENTENBESCHICHTETES MEDIZINPRODUKT
DISPOSITIF MÉDICAL À ÉLUTION MÉDICAMENTEUSE

(30) Priority: 12.07.2011 US 201161506811 P
(43) Date of publication of application: 21.05.2014
(73) Proprietor: Boston Scientific Scimed, Inc., Maple Grove, MN 55311-1566 (US)
(72) Inventor: FOLAN, Martyn, Loughrea Co. Galway (IE); HORGAN, Fergal, Shrule Co. Mayo (IE); TURKINGTON, Marie, Shrule Co. Mayo (IE)
(74) Representative: Peterreins Schley
(86) International application number: PCT/US2012/045260
(87) International publication number: WO 2013/009520

(56) References cited:
- WO-A2-98/39056
- US-A1- 2005 187 607
- US-A1- 2010 292 668
- US-A1- 2011 087 191

## Description

### TECHNICAL FIELD

This disclosure relates to medical devices for therapeutic agent delivery, and more particularly, to medical devices containing biodegradable polymer layers for therapeutic agent delivery.

### BACKGROUND

The body includes various passageways such as blood vessels (e.g., arteries) and lumens. These passageways sometimes become occluded (e.g., by a tumor or plaque). To widen an occluded vessel or lumen, balloon catheters can be used, e.g., in angioplasty.

A balloon catheter can include an inflatable and deflatable balloon carried by a long and narrow catheter body. The balloon can be initially folded around the catheter body to reduce the radial profile of the balloon catheter for easy insertion into the body.

During use, the folded balloon can be delivered to a target location in the vessel, e.g., a portion occluded by plaque, by threading the balloon catheter over a guide wire placed in the vessel. The balloon is then inflated, e.g., by introducing a fluid into the interior of the balloon. Inflating the balloon can radially expand the vessel so that the vessel can permit an increased rate of blood flow. After use, the balloon is typically deflated and withdrawn from the body.

US 2010/292668 A1 discloses an expandable balloon catheter for drug delivery, US2005187607 discloses a drug delivery device including a stent.

WO9839056 discloses a removable balloon protector.

### SUMMARY

The invention is defined as in the appended claims. Subject matter referred as embodiment(s) and/or invention(s) which are not claimed do not form part of the invention. Therapeutic agents can be delivered to the vessels and lumens of the body (body lumen) via medical devices, such as endoprostheses. The present disclosure is based, at least in part, on an endoprosthesis (e.g., a sleeve) that can be used to deliver therapeutic agents to *de novo* lesion sites. In some embodiments, one or more sleeves can be delivered to one or more body lumen sites in relatively few intervention procedures. In some embodiments, a sleeve can be used to deliver therapeutic agents to the site of a previously deployed stent, or a stent may be co-administered along with one or more sleeves (e.g., the sleeve may be disposed on an abluminal surface of the stent, the adluminal surface of the stent, or both).

Accordingly, in one aspect, the disclosure features a medical device as further defined in appended claim 1.

Embodiments of the above-mentioned medical devices can have one or more of the following features.

In some embodiments, one or more sleeve is biodegradable within a period of about 1 month to about 3 months. The matrix including a polymer can include any of the polymers described, *infra.* For example, the polymer can be selected from the group consisting of polyurethane, polyethylene, polylactic acid, polyglycolic acid, polylactic-co-glycolic acid, poly-DL-lactide, and any combination thereof.

The tissue-adhesive region can be configured on the abluminal surface as a plurality of strips, a plurality of dots, a continuous layer, a matrix mesh, a plurality of longitudinal strips, a plurality of circumferential strips, or any combination thereof. The tissue-adhesive region can include a repeating pattern of strips, dots, or both. The tissue adhesive region can include about 5 percent or more and/or about 95 percent or less of an abluminal surface area of the substrate layer of each sleeve. The tissue-adhesive region can include any of the tissue adhesive substances described, *infra.* For example, the tissue-adhesive region can include polyethylene glycol, dextran aldehyde, amino acid-based adhesives, adhesive surface proteins, microbial surface components-recognizing adhesive matrix molecules ("MSCRAMMS"), fatty ester modified PLA, fatty ester modified PLGA, gel particles, poly(N-isopropylacrylamide) gel particles, or any combination thereof.

The first release region can be adherent to the adluminal surface of the substrate layer of the second outer sleeve. A second release region can be disposed between the first inner sleeve and the abluminal surface of the expandable balloon. The second release region can be adherent to an abluminal surface of an expandable balloon. The release region can include, for example, contrast agents (e.g., iopromide), proteins (e.g., gelatin-based glues, protein-based adhesives), synthetic glues (e.g., cyanoacrylates), or any combination thereof.

The biologically active agent can include any of the biological active agents described, *infra.* For example, the biologically active agent can be selected from the group consisting of paclitaxel, everolimus, sirolimus, zotarolimus, and biolimus A9, and any combination thereof.

The medical device can further include one or more additional sleeve overlying the second sleeve. The substrate layers in the first and second sleeves can include the same or different material. In some embodiments, the medical device can include a vascular cuff.

In some examples, for the method of treatment, the first release region can elute within the body lumen after step (b). A second release region between the first sleeve and the expandable balloon can degrades into the body lumen after step (c). The method of treatment can further include rapidly degrading a body lumen adhered sleeve, such as by flushing a body lumen with saline solution, changing a pH, administering cryo-treatment, ultrasonicating, or combinations thereof. The medical device can be disposed over an expandable balloon. The body lumen can include a blood vessel or a bifurcated blood vessel or similar anatomical architecture.

In some examples, the method of making a medical device can further include (d) applying a second release agent over the first sleeve, (e) applying a second solution comprising a second polymer and a second biologically active agent to a non-stick substrate to form a second substrate layer; (f) applying tissue-adhesive portions to the second substrate layer to form a second sleeve; (g) removing the second sleeve from non-stick substrate; and/or (h) disposing the second sleeve over the second release agent-coated first sleeve to provide a medical device comprising a tubular assembly.

Embodiments of the above-mentioned medical devices can have one or more of the following advantages.

In some embodiments, the medical device is capable of delivering more than one drug. A plurality of devices can be arranged for use in multiple target lesions during a given intervention. The medical device can be relatively easily made by spraying, and/or dipping. The medical device can be scaled for peripheral or coronary interventions. For example, the medical device can be larger for peripheral vessels. In some embodiments, the medical device can be used for diffuse lesions, for bifurcated vessels, and/or for use in medical procedures that require bailout. In some embodiments, the medical device can be used without tertiary equipment, thereby providing cost benefits. In some embodiments, the medical device can minimize the overall clinical procedural time while reducing the requirement for additional interventional procedures. Examples of tertiary equipment and additional interventions include stenting or scenarios where multiple devices may be required for treatment of a vascular lesion.

The medical devices of the present disclosure include implantable and insertable medical devices that are used for the treatment of various mammalian tissues and organs. As used herein, "treatment" refers to the prevention of a disease or condition, the reduction or elimination of symptoms associated with a disease or condition, or the substantial or complete elimination of a disease or condition. Subjects are vertebrate subjects, more typically mammalian subjects including human subjects, pets and livestock.

As used herein, a "layer" of a given material is a region of that material whose thickness is substantially less than its length and width. Layers can be in the form of open structures (e.g., sheets, in which case the thickness of the layer is substantially less than the length and width of the layer), and partially closed structures (e.g., open tubes, in which case the thickness of the layer is substantially less than the length and diameter of tube).

As used herein, a polymer is "biodegradable" if it undergoes bond cleavage along the polymer backbone *in vivo,* regardless of the mechanism of bond cleavage (e.g., enzymatic breakdown, hydrolysis, oxidation, etc.). A biodegradable polymer includes "bioerosion" or "bioabsorption" of a polymer-containing component of a medical device (e.g., a polymer-containing layer), as well as other *in vivo* disintegration processes such as dissolution, etc. Biodegradability is characterized by a substantial loss *in vivo* over time (e.g., the period that the device is designed to reside in a patient) of the original polymer mass of the component. For example, losses may range from 50% to 75% (e.g., to 90%, to 95%, to 97%, to 99%, or more) of the original polymer mass of the device component. Bioabsorption times may vary widely, for example, bioabsorption times can range from several hours to approximately one year.

The details of one or more embodiments of the disclosure are set forth in the accompanying drawings and the description below. Other features, objects, and advantages of the disclosure will be apparent from the description and drawings, and from the claims.

### DESCRIPTION OF DRAWINGS

FIG. 1A is a side view of an embodiment of a medical device;
FIG. 1B is a cross-sectional view of an embodiment of a medical device;
FIG. 1C is an enlarged cross-sectional view of an embodiment of a medical device;
FIGS. 2A-2C are side views of an embodiment of a medical device during deployment;
FIGS. 3A-3C are cross-sectional views of an embodiment of a medical device in a body lumen;
FIGS 4A-4C are cross-sectional views of an embodiment of a medical device in a body lumen;
FIGS. 5A-5B are cross-sectional views of an embodiment of a medical device;
FIGS. 6A-6C are enlarged cross-sectional views of an embodiment of a medical device during deployment;
FIGS. 7A-7C are side views of an embodiment of a medical device during deployment;
FIGS. 8A-8B are side views of an embodiment of a medical device during deployment;
FIGS. 9A-9C show an example of a method of manufacture of a medical device; and
FIGS. 10A-10C show an example of a method of manufacture of a medical device.

Like reference symbols in the various drawings indicate like elements.

### DETAILED DESCRIPTION

In embodiments, this disclosure relates to a medical device (e.g., a vascular cuff or a sleeve) that can elute a therapeutic agent. The medical device can provide improved single or multiple delivery of a therapeutic agent to, for example, peripheral and/or cardiovascular body lumen walls. The medical device can be carried by an inflatable carrier balloon. When the balloon is inflated in a vascular lumen, the medical device can intimately contact the vasculature and adhere to the interior of a lumen's treatment site (e.g., endothelial cells lining the vasculature, atherosclerotic plaque at a targeted site). Upon subsequent balloon deflation and withdrawal, the vascular cuff remains at the treatment site.

The medical device (e.g., a sleeve) can provide drug delivery in a temporary capacity. For example, when a sleeve is degradable, the sleeve can decrease the likelihood of device-related thrombosis or embolism while providing drug treatment for vascular inflammation, and delayed re-endothelialization. In some embodiments, the sleeve can be used to treat sites where stent implantation is not desirable, such as small vessels, bifurcated lumen treatment sites, in-stent restenosis, and acute ST-elevation myocardial infarction. The sleeve can be used for *de novo* vascular lesions, where an unstented body lumen wall has lesions, calcified or otherwise. The sleeve can be used for secondary treatment at locations where restenosis has formed. The sleeve can be used instead of or in addition to drug-eluting stents. In some embodiments, the sleeve can homogenously deliver therapeutic agents (e.g., an anti-restenotic agent such as paclitaxel, everolimus, rapamycin, biolimus, zotarolimus, etc.) to a target lesion, which can decrease the likelihood of vascular stiffening, while maintaining the accessibility of the blood vessel to re-intervention and decreasing the likelihood of restenosis, when compared to conventional interventions (e.g., stent implantation, balloon angioplasty).

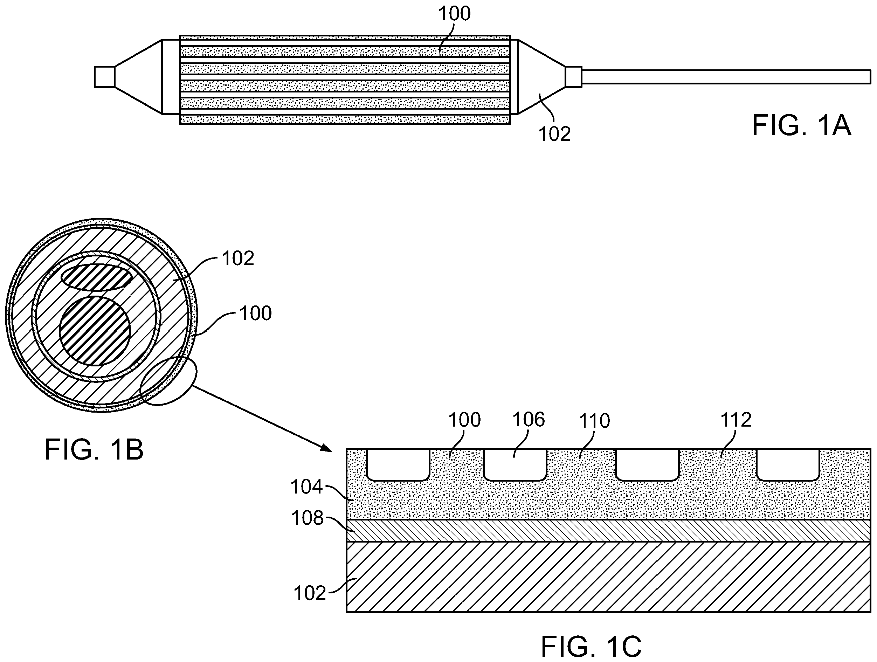

Referring to FIG. 1A, the medical device has a tubular construction, in the form of a sleeve 100 (e.g., a vascular cuff). Sleeve 100 can be used in conjunction with a carrier balloon expandable catheter 102 and can be left within a blood vessel following balloon deployment and withdrawal. The sleeve can deliver therapeutic agents during or in addition to angioplasty procedures, and can provide prolonged drug delivery to a body lumen wall after angioplasty procedures. In some embodiments, the sleeve can provide one or more therapeutic agents, which can elute over specific time frames and/or in particular sequences. The sleeve can decrease the likelihood of drug loss from a body lumen wall, or the premature drug loss from a drug delivery device, for example, when a drug delivery device is maneuvered through a blood vessel.

Referring to FIGS. 1B and 1C, a sleeve 100 can include a biodegradable substrate layer 104 and one or more tissue adhesive region(s) 106 on an abluminal surface of the sleeve. One or more balloon release region(s) 108 can be disposed on an adluminal surface of the sleeve, between sleeve 100 and balloon 102. Biodegradable substrate 104 can provide structural shape to the sleeve, and a matrix 110 (e.g., a polymeric matrix) which can contain a therapeutic agent 112. Biodegradable substrate layer 104 can protect the therapeutic agent, for example, until a target treatment site is reached. The polymer matrix 110 can elute the therapeutic agent in a controlled manner. After drug elution is completed during a predetermined time frame, biodegradable substrate layer 104 can degrade in a controlled manner leaving little or no residual components at the treatment site.

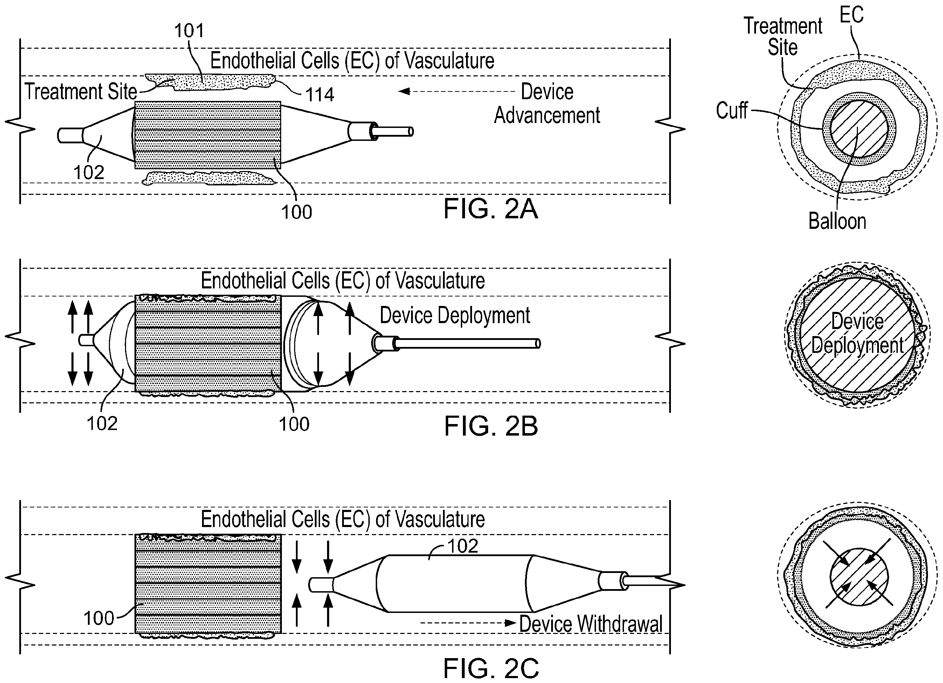

Referring to FIGS. 2A-2C, sleeve 100 is administered using a balloon carrier 102 in a target area 101 of a body lumen. The balloon can be inserted into a target area (FIG. 1A), inflated to adhere the sleeve to the target area (FIG. 1B), then deflated and withdrawn from the target area (FIG. 1C) while leaving the sleeve in the body lumen. In some embodiments, when implanted in a body lumen, the sleeve is biodegradable within a period of two weeks or more (e.g., one month or more, six weeks or more, or two months, or more) to three months or less (e.g., two months or less, six weeks or less, or one month, or less). In some embodiments, the sleeve can degrade within a period of about several minutes or more (e.g., about two minutes or more, about five minutes or more, about ten minutes or more, about 20 minutes or more, about an hour or more, about five hours or more, about 12 hours or more, or about 24 hours, or more). The biodegradability of the sleeve can depend on the polymers of the biodegradable substrate layer, the tissue adhesive region, and the therapeutic agent.

For example, in a sleeve including PLGA, the copolymer ratio of lactide to glycolide can determine the rate of polymer degradation, where the higher the lactide content, the slower the degradation. In some embodiments, molecular weight can affect the degradation, where the lower the molecular weight, the faster the degradation (when the molecular weight is below the range where T_{g} is affected). In some embodiments, a polymer end group can be used to control the rate of degradation. For example, an alkyl end group associated with a co-polymer, such as PDLLA, can result in slower degradation than a polymer with an acid end group, such as in PLGA. In some embodiments, factors such as crystallinity, drug percent loading, and/or other additives can also affect degradation. For example, the addition of a therapeutic agent such as a hydrophobic drug (e.g., everolimus or paclitaxel) can be used to delay the rate of molecular weight loss.

In some embodiments, for thicknesses of less than or equal to about 200 µm, sleeve thickness can have minimal impact on degradation. In some embodiments, for thicknesses of greater than about 200 µm, oligomers diffuse out of a polymer layer at a relatively slow rate, resulting in an accumulation of acidic molecular weight degradation products at the center of the material, which can cause autocatalytic degradation (e.g. heterogeneous degradation). As an example, *in vitro* mass loss in bio-relevant media at 37° for a film of about 200 µm thick including 85/15 lactide : glycolide PLGA co-polymer can demonstrate greater than 85% mass loss in less than about 180 days.

Matrix materials which may be used to form biodegradable substrate layers include synthetic and natural biodegradable polymers. Synthetic biodegradable polymers include polyesters, for example, selected from homopolymers and copolymers of lactide, glycolide, and epsilon-caprolactone, including poly(L-lactide), poly(D, L-lactide), poly(lactide-co-glycolides) such as poly(L-lactide-co-glycolide) and poly(D, L-lactide-co-glycolide), polycarbonates including trimethylene carbonate (and its alkyl derivatives), polyphosphazines, polyanhydrides, polyorthoesters, and biodegradable polyurethanes. Natural biodegradable polymers include proteins, for example, selected from fibrin, fibrinogen, collagen and elastin, and polysaccharides, for example, selected from chitosan, gelatin, starch, and glycosaminoglycans such as chondroitin sulfate, dermatan sulfate, keratin sulfate, heparin, heparan sulfate, and hyaluronic acid. In some embodiments, the polymers can include one or more of alginate, dextran, chitin, cotton, polylactic acid-polyethylene oxide copolymers, cellulose, and chitins. Blends of the above natural and synthetic polymers may also be employed.

In some embodiments, polymers suitable for biodegradable substrate layers can include without limitation polyurethane and its copolymers, silicone and its copolymers, ethylene vinyl-acetate, polyethylene terephthalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polycarbonate, poly(glycolide-lactide) copolymer, Tecothane, PEBAX, polyethylene, polylactic acid, poly(K-caprolactone), poly(K-hydroxybutyrate), polydioxanone, poly(K-ethyl glutamate), polyiminocarbonates, poly(ortho ester), and/or polyanhydrides. Additional polymeric materials are described, for example, in U.S. Patent No. 5,650,234 and 5,463,010. Blends of the above polymers may also be employed.

In some embodiments, biodegradable substrate layer 104 includes biodegradable materials, such as polyglycolic acid, polylactic acid, poly(lactic-co-glycolic acid), poly-DL-lactide, and/or other known degradable polymers. Biodegradable substrate layer 104 can also include non-biodegradable materials, such as Tecothane, PEBAX, and/or polyethylene. In some embodiments, biodegradable substrate layer 104 contains, for example, from 1 to 100 wt% (e.g., from about 25 to about 50 wt %, from about 25 to about 75 wt %, from about 75 to about 90 wt%, from about 85 to about 99 wt %, from about 90 to about 99 wt %, from about 95 to about 99 wt%, 100 wt%) of one or more biodegradable polymers. In some embodiments, the weight percent of biodegradable material can be about 80 % or more (e.g., about 90 % or more, about 95 % or more, or about 99 % or more) of the total polymer contained by biodegradable substrate layer 104. The weight percent of non-biodegradable material can be about 20 % or less (e.g., about 10 % or less, about 5 % or less, or about 1 % or less) of the total polymer contained by biodegradable substrate layer 104. In some embodiments, incorporation of a non-biodegradable material can provide increased stability to the resulting material, such that the biodegradable substrate layer can have increased resistance to degradation (e.g., during storage, in humid environments). In some embodiments, biodegradable substrate layer 104 can be in the form of a fibrous scaffold with an open porous structure that encourages three-dimensional migration and proliferation of cells within the fibrous scaffold. Examples of biodegradable substrate layer 104 include non-porous layers and porous layers.

Biodegradable substrate layer 104 can have any dimension that functions as described herein. For example, in some embodiments, biodegradable substrate layer 104 can have a thickness of about 5 nm or more (e.g., about 10 nm or more, about 20 nm or more, about 50 nm or more, about 100 nm or more, about 500 nm or more, about one micron or more, about 10 microns or more, about 25 microns or more, about 50 microns or more, or about 70 microns or more) and/or about 80 µm or less (about 70 microns or less, about 50 microns or less, about 25 microns or less, about 10 microns or less, about one micron or less, about 500 nm or less, about 100 nm or less, about 50 nm or less, about 20 nm or less, or about 10 nm or less). In some embodiments, biodegradable substrate layer 104 can be a uniform layer or patches that may or may not be interconnected. The biodegradable substrate layer can define the length and expanded diameter of the sleeve. For example, the biodegradable substrate layer can have a length of about four mm or more (e.g., about ten mm or more, about 20 mm or more, about 30 mm or more, about 40 mm or more, or about 50 mm or more) and/or about 60 mm or less (e.g., about 50 mm or less, about 40 mm or less, about 30 mm or less, about 20 mm or less, or about ten mm or less).). In some embodiments, the biodegradable substrate layer can have an expanded diameter of about 2 mm or more (e.g., about 3 mm or more, about 4 mm or more, or about 5 mm or more) and/or about 6 mm or less (e.g., about 5 mm or less, about 4 mm or less, or about 3 mm or less).

In some embodiments, tissue-adhesive region 106 can include one or more tissue-adhesive substances. The tissue-adhesive substances can be provided in biodegradable substrate layer 104 (e.g., evenly dispersed in the layer or having a higher concentration at a tissue contacting surface of the layer). In some embodiments, one or more adhesive substances can be provided in an adhesive region that is disposed over the surface of biodegradable substrate layer 104 (which adhesive region may penetrate the biodegradable substrate layer to a certain degree). For example, a pure layer of an adhesive substance or a layer containing an adhesive substance and a suitable excipient may be applied to a tissue contacting surface of a biodegradable substrate layer. The tissue-adhesive region allows the sleeve to be in close proximity to the vasculature, reducing the potential for blood leakage from the sleeve into the body lumen while providing therapeutic agent delivery to a vascular treatment site.

In some embodiments, the tissue-adhesive region can be configured as a plurality of strips, a plurality of dots, a continuous layer, a matrix mesh, a plurality of longitudinal strips, a plurality of circumferential strips, or any combination thereof. The tissue-adhesive region can include a repeating pattern of dots and/or strips at predetermined locations. In some embodiments, the tissue adhesive region is disposed over about 5 % or more (e.g., about 10 % or more, about 15 % or more, about 20 % or more, about 25 % or more, about 30 % or more, about 50 % or more, about 75 % or more, or about 90 % or more) and/or about 95 % or less (about 90 % or less, about 75 % or less, about 50 % or less, about 30 % or less, about 25 % or less, about 20 % or less, about 15 % or less, or about 10 % or less) of the abluminal surface area of the biodegradable substrate layer that the tissue-adhesive region is disposed on. In some embodiments, the tissue adhesive region can cover greater than 0 % up to 100 % of the surface area of an immediately underlying biodegradable substrate layer. The tissue adhesive region can be porous. In some embodiments, when the tissue adhesive region covers 100 % of the underlying biodegradable substrate layer, the adhesive region can protect a therapeutic agent until a treatment site is reached. The surface area of the tissue adhesive region can be dependent on the adhesive properties. In some embodiments, the tissue adhesive region does not delay drug elution from the sleeve.

In some embodiments, the tissue-adhesive region can have a thickness of about ten nm or more (e.g., about 20 nm or more, about 30 nm or more, about 40 nm or more, about 50 nm or more, about 60 nm or more, about 70 nm or more, about 80 nm or more, or about 90 nm or more) and/or about 100 nm or less (about 90 nm or less, about 80 nm or less, about 70 nm or less, about 60 nm or less, about 50 nm or less, about 40 nm or less, about 30 nm or less, or about 20 nm or less). In some embodiments, the tissue - adhesive region thickness can be influenced by the choice of adhesive. For example, a protein-based adhesive layer can be in the form of a chain of amino acids (a thickness of less than about 10 nm) or can have a thickness that is as large as the sub-micron based poly(N-isopropylacrylamide) gel particles.

A tissue adherent strength of a material can be assessed through *in vitro* based peel tests and nano-indentation, typically used to measure the interfacial adhesive properties. For example, nano-indentation data (indicative of Youngs modulus, hardness) can be used to correlate thickness with adhesive properties. In some embodiments, the lap-shear strength of a given adhesive can be evaluated to comply with values reported for typical soft-tissue adhesives (about 15 to about 45 kPa).

Tissue-adhesive region can include bioadhesive materials, such as natural polymeric materials, synthetic materials, and synthetic materials formed from biological monomers such as sugars. Tissue adhesives can also be obtained from the secretions of microbes, marine mollusks, and crustaceans. The tissue adhesives can have better adhesion to body tissue, and can have better adhesion to the abluminal surface of a sleeve that the adhesive is attached to rather than to the adluminal surface of an overlying sleeve, or to an overlying release region. In other words, the adhesion at the interface of the sleeve and the carrier balloon (or at the interface of the sleeve and an adjacent stacked sleeve) is weaker than the adhesion at the interface of the biodegradable substrate layer and the tissue adhesive disposed thereon (or at the interface of the tissue adhesive and the body tissue) so that the biodegradable substrate layer remains with the tissue adhesive region when the carrier balloon is retracted from the body.

In some embodiments, the tissue-adhesive region includes an adhesive material such as polyethylene glycol, dextran aldehyde, amino acid-based adhesives, adhesive surface proteins, microbial surface components recognizing adhesive matrix molecules ("MSCRAMMs"), fatty ester modified PLA, fatty ester modified PLGA, gel particles, and/or poly(N-isopropylacrylamide) gel particles.

As an example, a polar molecule may be employed as an adhesive material for the adhesive region. Examples of such polar molecules include poly(amino acids). For instance, in some embodiments, an amphipathic poly(amino acid) is used as an adhesive material. The amphipathic poly(amino acid) may have a hydrophobic poly(amino acid) tail (e.g., ranging from 2 to 400 or more amino acids in length) to encourage interaction with the lesion. Examples of hydrophobic amino acids include phenylalanine, leucine, isoleucine and valine, among others. The amphipathic poly(amino acid) may have a hydrophilic poly(amino acid) head (e.g., ranging from 2 to 400 or more amino acids in length) to encourage interaction with the biodegradable polymer (where a hydrophilic polymer such as hyaluronic acid is employed). Examples of hydrophilic amino acids include basic amino acids (e.g., lysine, arginine, histidine, ornithine, etc.), acidic amino acids (e.g., glutamic acid, aspartic acid, etc.), and neutral amino acids (e.g., cysteine, asparagine, glutamine, serine, threonine, tyrosine, glycine). The hydrophilic poly(amino acid) head can be zwitterionic to promote ion-dipole bonding with the biodegradable polymer (where a hydrophilic polymer such as hyaluronic acid is employed). Such a polymer head can contain a mixture of acidic (anionic) and basic (cationic) amino acids and may range, for example, from 2 to 400 or more amino acids in length.

A poly(amino acid) containing a cell-binding peptide such as YIGSR or RGD can be employed as an adhesive material for the adhesive region. Such sequences can be repeated if desired. The poly(amino acid) may further comprise a hydrophilic poly(amino acid) chain (e.g., typically ranging from about 2 to about 400 or more amino acids in length) to promote interaction with the biodegradable polymer (where a hydrophilic polymer such as hyaluronic acid is employed).

In some embodiments, the amino acid 3,4-dihydroxyphenyl alanine (DOPA) or a poly(amino acid) chain that includes multiple DOPA units can be used as an adhesive substance for the adhesive region. Such chains may further include lysine units, along with the DOPA units. See Statz et al. J. Am. Chem. Soc. 127, 2005, 7972-7973, wherein a 5-mer anchoring peptide (DOPA-Lys- DOPA-LysDOPA) was chosen to mimic the DOPA- and Lys-rich sequence of a known mussel adhesive protein.

In some embodiments, MSCRAMMs (microbial surface components recognizing adhesive matrix molecules) are employed as adhesive substances. Examples of MSCRAMMs include fibronectin binding proteins (e.g., FnBPA, FnBPB, etc.) and fibrinogen binding proteins (e.g., ClfA, ClfB, etc.), among others. See, e.g., Timothy Foster, Chapter 1, "Surface protein adhesins of staphylococci," from Bacterial Adhesion to Host Tissues: Mechanisms and Consequences, Edited by Michael Wilson, 2002, pages 3-11.

In some embodiments, because plaque lesions are known to be hydrophobic, a hydrophobic drug (e.g., paclitaxel, among many others) can be provided over or within the biodegradable polymer containing layer, encouraging adhesion and/or uptake by the lesion upon contact with a lesion.

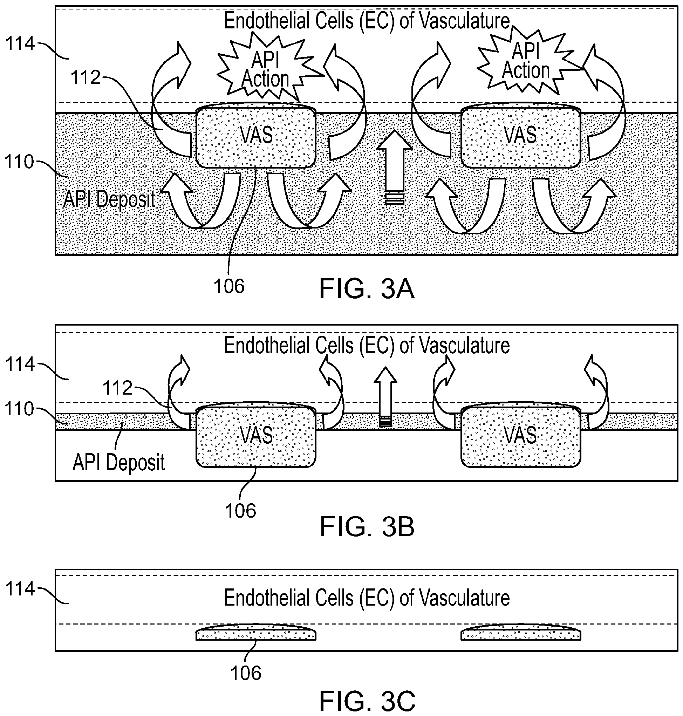

Referring to FIGS. 2A-2C, tissue adhesive region 106 can cause an interference fit (e.g., a mechanical interaction) between the sleeve 100 and an inner surface 114 of the vascular wall to which it contacts, thereby retaining the sleeve at the target area. Referring to FIGS. 3A-3C, tissue adhesive region 106 can improve the therapeutic agent's local proximity, and/or reduce a required dosage of a therapeutic agent to the target treatment area's cellular lining 114, for example, by decreasing therapeutic agent wash-off to the body lumen. In some embodiments, therapeutic agent 112 can diffuse out of the sleeve to be absorbed by a vessel wall and the diffusion rate can be controlled by a therapeutic agent concentration within the sleeve and the substrate properties. For example, the ratio of polymer to therapeutic agent can influence the porosity of the sleeve and affect the ability of the therapeutic agent to diffuse out of the matrix. A greater ratio of therapeutic agent can increase the porosity of the sleeve and increase therapeutic agent diffusion. Thus, the therapeutic agent can elute into a blood vessel wall after implantation of the sleeve over a predetermined duration. Once the drug has finished eluting, the sleeve can degrade to reduce the likelihood of adverse biological reactions (e.g., embolic formation) and to return a vascular lining to its native condition. In some embodiments, referring to FIG. 3C, tissue adhesive region 106 can remain within the body lumen after the clinical procedure is completed. In some embodiments, tissue adhesive region 106 can substantially degrade (e.g., degrade by about 80 wt% or more, degrade by about 90 wt% or more, degrade by about 95 wt% or more) before the complete degradation of layer 104, or can substantially degrade after layer 104 has completely degraded.

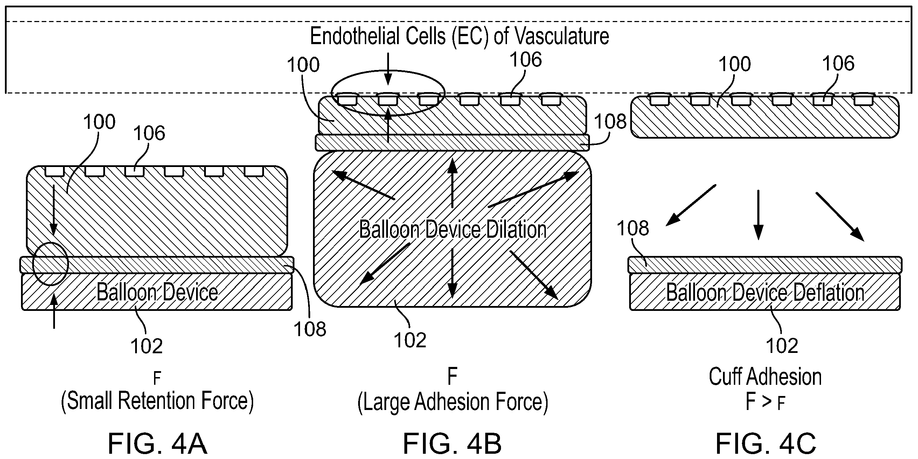

In some embodiments, tissue adhesive region 106 can include hydrogels (e.g., polyethylene glycol:dextran aldehyde) to allow for a strong attractive force to the inner surface of a blood vessel. In some embodiments, referring to FIGS. 4A-4C, the attractive force between tissue adhesive region 106 and a lumen wall tissue is greater than the retention force between the adluminal surface of the sleeve and the balloon's outer surface, such that the dilation and pressurization of tissue-adhesive region 106 to lumen's inner surface (e.g., endothelial cell layer, a vascular plaque) sever the retention force between sleeve 100 and balloon 102. Subsequent to balloon deflation and withdrawal, the tissue-adhesive region 106 retains the vascular cuff in intimate contact with the vasculature. The retention force between sleeve 100 and balloon 102 can result from chemical adhesive forces (e.g., exerted by release region 108) or physical forces (e.g., frictional forces between sleeve 100 and carrier balloon 102).

Referring back to FIG 1C, balloon 102 can be coated in part or in full with one or more balloon release region(s) 108. Balloon release region 108 can help retain sleeve 100 on balloon 102, which is loaded onto the balloon catheter. Balloon release region can be temporary and biocompatible. For example, balloon release region 108 can include formulations of a contrast agent, such as iopromide (Ultravist ®), which can be used as a contrast medium and as balloon adhesive. In some embodiments, the release region can be configured as a plurality of strips, a plurality of dots, a continuous layer, matrix mesh, a plurality of longitudinal strips, a plurality of circumferential strips, or any combination thereof. The release region can include a repeating pattern of dots and/or strips, which can be at predetermined locations. In some embodiments, the release region are disposed over about 5 % or more (e.g., about 10 % or more, about 15 % or more, about 20 % or more, about 25 % or more, about 30 % or more, about 50 % or more, about 75 % or more, or about 90 % or more) and about 95 % or less (e.g., about 90 % or less, about 75 % or less, about 50 % or less, about 30 % or less, about 25 % or less, about 20 % or less, about 15 % or less, or about 10 % or less) of the abluminal surface area of an underlying balloon or sleeve. In some embodiments, the release region can cover greater than 0% up to 100 % of the surface area of an underlying balloon or sleeve. The release region can be porous. The surface area of the release region can be dependent on its adhesive and degradation properties. As an example, in some embodiments, specimens including a total surface area of about 0.4 cm² of a gelatin-based biomimetic adhesive can generate adhesive strengths of 12-23 kPa. Therefore, the adhesive strength can be appropriately adjusted by modulating the extent of the contact surface area.

In some embodiments, the release region can have a thickness of about ten nm or more (e.g., about 20 nm or more, about 30 nm or more, about 40 nm or more, about 50 nm or more, about 60 nm or more, about 70 nm or more, about 80 nm or more, or about 90 nm or more) and/or about 100 nm or less (about 90 nm or less, about 80 nm or less, about 70 nm or less, about 60 nm or less, about 50 nm or less, about 40 nm or less, about 30 nm or less, or about 20 nm or less).

In some embodiments, the release region can include contrast agents (e.g., iopromide), proteins (e.g., gelatin-based glues, protein-based adhesives), synthetic glues (e.g., cyanoacrylates), or any combination thereof. For example, the release region can include gelatin-based glues (e.g., resorbable biological glues such as GRFG - gelatin, resorcinol, formaldehyde, glutaraldehyde), gelatin hydrogel glues, cyanoacrylates (e.g. Histoacryl blue), adhesive based on protein engineering (e.g., high grade biocompatibility and biodegradability internal adhesives). In some embodiments, for better retention of the release region on a balloon surface or on an underlying sleeve surface during delivery, the release region can include crosslinked gel particles, or the gel particles can be mixed with a higher molecular weight polymer.

In some embodiments, the balloon surface can have "windows" that can allow for release of a physico-mechanical signal across the "window" to facilitate sleeve detachment. A "window" can include a hole, aperture, a pore, a thinner area of the same polymer, and/or a membrane of an alternate material. For example, the windows can enable the transfer of a detachment agent (e.g., a change in temperature, a change in pH) across the window when the deployment balloon has been flushed with an appropriate catalyst. The catalyst can include an external agent, which can be physical or chemical in nature. As an example, a cryo-technique such as that used in the cryo-catheter devices can deliver extreme cold (a catalyst) from the tip of an ablation catheter or through a balloon. Similarly, heat (a catalyst) can be applied in this manner. In some embodiments, adhesion can be regulated through modulation of pH. For example, the availability of local calcium ions (a catalyst) can be adjusted and used to vary alkaline balance.

The catalyst can initiate localized site degradation of a window, when the window is, for example, a thinner area of the same polymer or of a membrane of an alternate material. In some embodiments, the catalyst can initiate the degradation of a balloon adhesive to allow a sleeve to be detached and deployed at a treatment site. The catalyst can be released through, for example, a hole, an aperture, a pore, a thinner area of the same polymer, or a membrane of an alternate material.

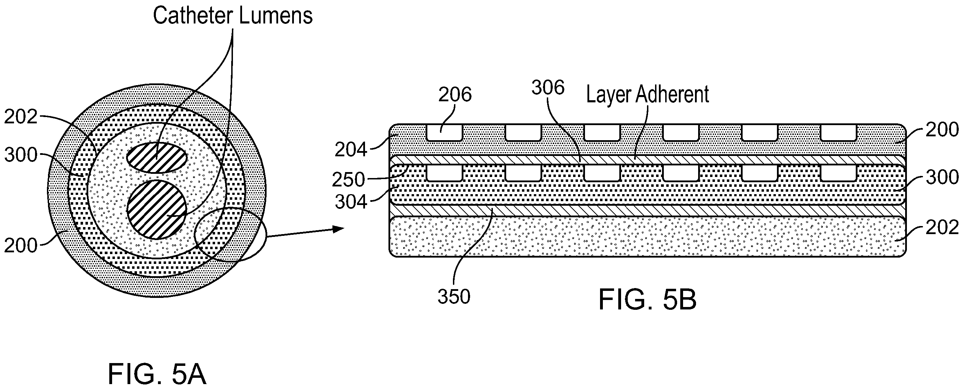

In some embodiments, multiple sleeves are arranged in a stacked configuration, where a relatively outer sleeve circumferentially overlies a relatively inner sleeve. When arranged in a stacked configuration, multiple sleeves can be delivered to multiple lumen locations during a given intervention. In some embodiments, referring to FIGS 5A-5B, multiple sleeves are arranged in a stacked configuration. Referring to FIG.5B, an outer sleeve 200 can have a biodegradable substrate layer 204 and abluminal tissue-adhesive region 206. The outer sleeve 200 overlies an inner sleeve 300, which in turn can have a biodegradable substrate layer 304 and abluminal-tissue adhesive region 306. Inter-sleeve release region 250 can exist between outer sleeve 200 and inner sleeve 300. A balloon-release region 350 can exist between an innermost sleeve (e.g., sleeve 300) and a carrier balloon 202. In some embodiments, the attractive force between adhesive region 206 and a lumen's inner surface (e.g., adluminal surface) is greater than the retention force between outer sleeve 200 and inner sleeve 300; and the adhesive force between the tissue adhesive region 306 and a lumen's inner surface is greater than the retention force between the innermost sleeve and the carrier balloon. The retention force between outer sleeve 200 and inner sleeve 300 can include chemical adhesive forces (e.g., exerted by layer 250) or physical forces (e.g., frictional forces between sleeves 200 and 300). Similarly, the retention force between inner sleeve 300 and balloon 202 can result from chemical adhesive forces (e. g., exerted by layer 350) or physical forces (e.g., frictional forces between sleeve 300 and carrier balloon 202).

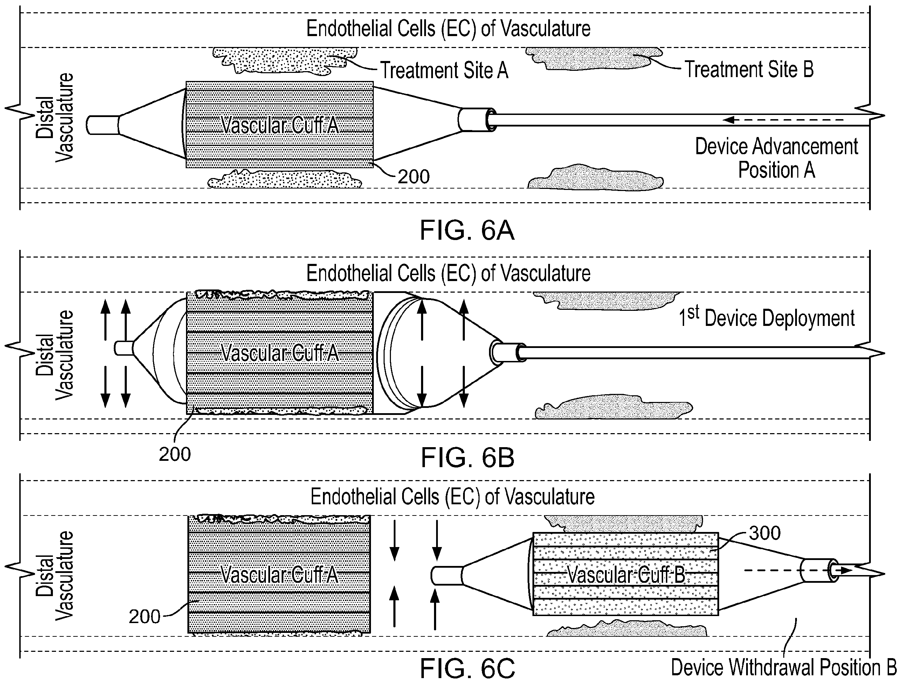

Referring to FIGS. 6A-6C, during deployment, first outer sleeve 200 can be applied to a treatment site upon balloon expansion. The balloon can then be deflated and advanced to a second treatment site, where a second inner sleeve 300 can be applied to a second treatment site upon balloon expansion. Stacked configurations having greater than two sleeves can be delivered to multiple treatment sites in an analogous manner. The stacked sleeves can be delivered in a controlled manner to sequential portions of the vasculature in a minimum amount of intervention procedures (e.g., a single intervention). A stacked sleeve configuration can be advantageous for diffuse lesion treatment, where a treatment area is not well defined. In some embodiments, a stacked configuration can be used for tapered body lumens, where multiple sleeves can be placed within the body lumen with minimal re-intervention (e.g., no re-intervention).

The stacked sleeves can each have different compositions. For example, depending on the composition of the biodegradable substrate layer, the tissue-adhesive region, and the therapeutic agent carried by each of the sleeves, the sleeves can degrade at different durations and can delivery different therapeutic agents to various wall regions of a body lumen. In some embodiments, inter-sleeve release and/or balloon-release region can each include one or more release substances. The release substances can be provided in the biodegradable substrate layer (e.g., evenly dispersed in the layer or more preferably having a higher concentration at a delivery vehicle contacting surface of the layer).

In some embodiments, one or more release substances can be provided in a release region that is disposed between the surfaces of the carrier balloon (or an underlying sleeve) and the biodegradable substrate layer of a given sleeve (which release region may penetrate the degradable substrate layer to a certain degree). One example of a release substance is zwitterionic phosphorylcholine and its derivatives. Phosphorylcholine is able to form ionic-dipole bonds with various polar substances, including biodegradable polymers such as hyaluronic acid and polar balloon materials such as PEBAX. In this way phosphorylcholine may act to bind the biodegradable polymer portion of the sleeve to the balloon material. When desired, a wetting agent (e.g., saline or water) can be employed to disrupt the ionic-dipole interactions holding the sleeve on the balloon.

In some embodiments, the wetting agent is supplied by the delivery vehicle (e.g., a delivery balloon). For instance, an inflatable micro-porous or weeping balloon may be used to dilate the vessel site and deliver a wetting agent which interacts with the zwitterionic phosphorylcholine. As another example, saline loaded microspheres may be provided between the biodegradable polymer containing layer and the balloon, which burst and release their contents upon balloon inflation.

Other zwitterionic materials may be employed as release substances including zwitterionic peptides. For example, peptides with both basic amino acids (e.g., lysine, arginine, ornithine, etc.) and acidic amino acids (e.g., glutamic acid, aspartic acid, etc.) will have zwitterionic character for providing ionic ionic-dipole bonds with various polar substances (e.g., a hydrophilic biodegradable polymer or a hydrophilic balloon material). Chains of non-polar amino acid chains (e.g., phenylalanine, leucine, isoleucine, valine, etc.) may be attached to zwitterionic chains for providing hydrophobic interactions with various nonpolar substances (e.g., a hydrophobic balloon material).

Shear sensitive adhesives constitute another class of release substance that may be used between a balloon delivery vehicle and a sleeve. The basic principle of these adhesives is that the shearing force that is created between the inflating balloon and the adhesive will break the bond and facilitate release. An example of such an adhesive is a blend of polyvinylpyrrolidone (PVP) and polyethylene glycol (PEG), which would provide a biocompatible layer which adheres the balloon to the biodegradable substrate layer until the device is in place at the delivery site. Balloon dilation may be used to disrupt the adhesive bonds and the sleeve may thus be released from the balloon. The weight ratio of PVP to PEG in such blends may vary widely, for example, ranging from 1:99 to 10:90 to 25:75 to 50:50 to 75:25 to 90:10 to 95:5 to 99:1.

In some embodiments, a release region degrades within the body lumen after implantation of an overlying sleeve. The release region degradation can be rapid, such that it can be completed before delivery of an underlying sleeve (e.g., when the release region adheres to an underlying sleeve after implantation of an overlying sleeve). In some embodiments, the release region can remain attached to the adluminal surface of an implanted overlying sleeve and can degrade with the overlying sleeve in a body lumen.

Degradation of the release region can occur in a controlled manner. For example, degradation of the release region can be completed in about three months or less (e.g., about two months or less, about one month or less, about two weeks or less, about one week or less, about three days or less, about two days or less, about one day or less, about twelve hours or less, about six hours or less, about one hour or less, about 30 minutes or less, about 15 minutes or less, about 5 minutes or less, or about one minute or less) and/or about 30 seconds or more (e.g., about one minute or more, about 5 minutes or more, about 15 minutes or more, about 30 minutes or more, about one hour or more, about six hours or more, about twelve hours or more, about one day or more, about two days or more, about three days or more, about one week or more, about two weeks or more, about one month or more, or about two months or more). This degradation profile can be designed to be of short duration if a release region has already fulfilled its primary function. In some embodiments, the release region degradation can be matched to the duration of the sleeve degradation.

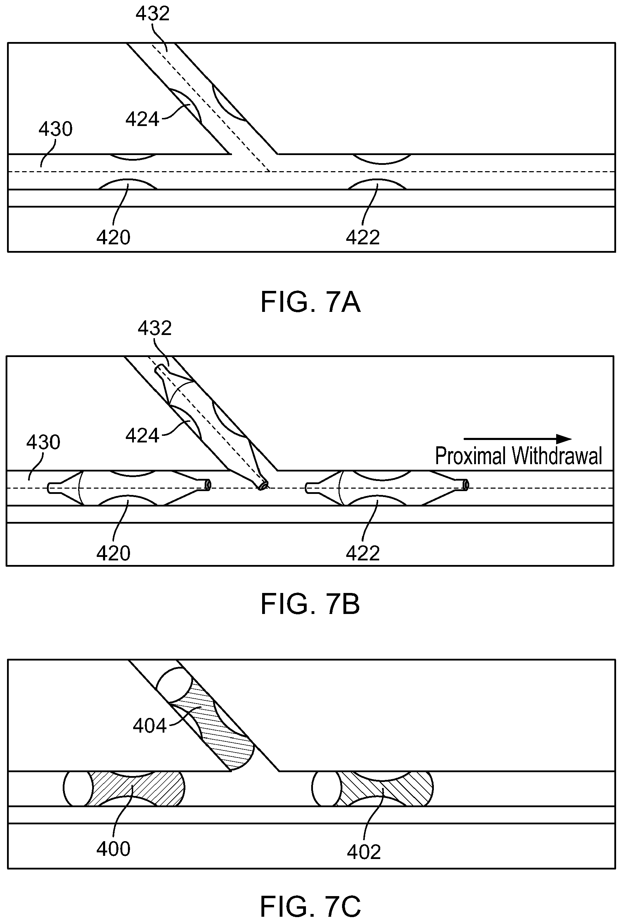

In some embodiments, when multiple sleeves are in a stacked configuration, the stacked sleeves can be used for bi-furcated treatment sites. Referring to FIG. 7A, a modeled bifurcated vasculature with three associated lesions 420, 422, and 424 is shown with two guidewire placement locations 430 and 432. Using three stacked sleeves 400, 402, and 404, the three lesion sites can be treated using a single interventional device as illustrated in FIGS. 7B and 7C. With a guidewire at location 422, the most proximal treatment site can be accessed and treated first with the balloon to leave the outermost sleeve 402 at treatment site 422. The balloon can now be advanced to the treatment site 420 and a repeat inflation/deflation can be performed to leave the second sleeve 400 at treatment site 422. Finally, the guide wire can be partially withdrawn and replaced at guide wire position 424 with a further inflation/deflation of the balloon to leave the innermost sleeve 404 at site 424. After placement of the sleeves, the balloon can be fully withdrawn. What remains after treatment (as shown in FIG. 7C) are the three sleeves at the appropriate vascular locations. The sleeves can deliver a predetermined therapeutic agent and therapeutic agent dosage at each of the treatment sites.

In some embodiments, the sleeve can also offer an opportunity for bailout. For example, if unforeseen malposition or vascular blockage is caused by a sleeve during use, the introduction of an appropriate bolus of suitable fluid to the locality of the problematic sleeve can be used to accelerate its degradation and thus return a blood vessel to its unblocked state. Treatment can be therefore administered without full interventional procedures (e.g., surgical intervention). Without wishing to be bound by theory, it is believed that malposition or vascular blockage can have an increased risk of occurrence in procedures involving distal vasculature with small lumen diameter. In some embodiment, depending on the composition of the sleeve, accelerated degradation of a sleeve can include flushing a body lumen with a saline solution, changing the pH of the local environment of a sleeve, administering cryo-treatment to the local environment of a sleeve, and/or administering ultrasound to the local environment of a sleeve. Accelerated degradation can occur over the period of about one month or less (e.g., about three weeks or less, about two weeks or less, about one week or less, about three days or less, about one day or less, about 12 hours or less, about six hours or less, about one hour or less, about 30 minutes or less, about 15 minutes or less, about five minutes or less, or about one minute or less).

As an example, a sleeve can include a 200 µm thick film of about 85/15 lactide: glycolide PLGA co-polymer, and *in vitro* mass loss tests can be conducted at 37 °C in bio-relevant media. Greater than 85 % mass loss of the film can occur in less than 180 days. As another example, a sleeve can include a 200 µm thick film of 50/50 lactide:glycolide PLGA co-polymer, and in vitro mass loss studies can be conducted at 37 °C in bio-relevant media. Greater than 90% mass loss of the film can occur in less than 145 days. Preclinical studies support these findings.

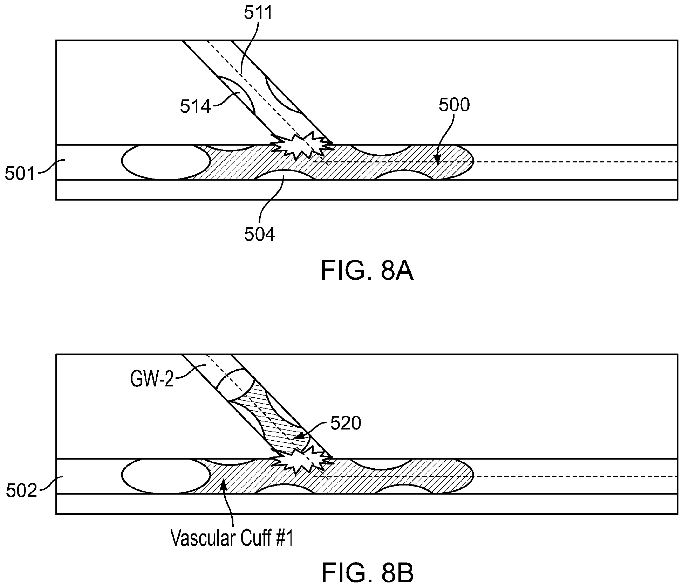

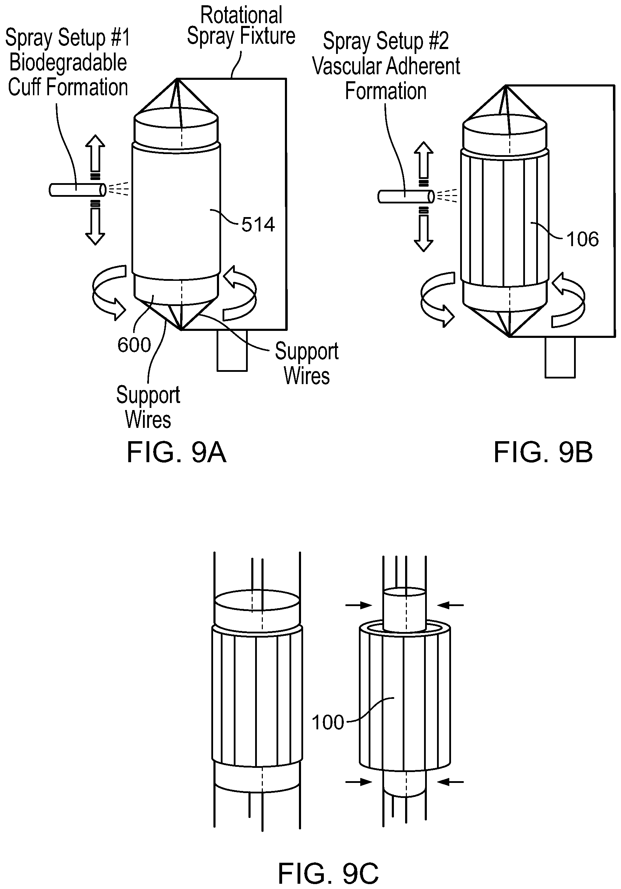

In some embodiments, referring to FIGS. 8A-8B, sleeves can be used to treat a bifurcated treatment site, post-jailing. For example, a primary body lumen 501 can be treated with an appropriately sized primary sleeve 500, which is used to span the length Li of the diffuse lesions 502 at treatment site 504. The bifurcated secondary lumen 511 is effectively cut off. The guidewire can be proximally withdrawn and rerouted into the bifurcation, through the already placed primary sleeve 500, and the secondary lesion site 514 in the bifurcation may be treated with a second sleeve 520 as shown in FIG. 9B. The sleeve can include a soft polymer matrix system that is readily breachable with the guide wire tip to allow bifurcation access.

A wide variety of therapeutic agents may be used in the sleeves. A therapeutic agent may be used singly or in combination with other therapeutic agents. The terms "therapeutic agent", "pharmaceutically active agent", "pharmaceutically active material", "pharmaceutically active ingredient", "drug", "beneficial agent", "bioactive agent" and other related terms may be used interchangeably herein and include, but are not limited to, small organic molecules, peptides, oligopeptides, proteins, nucleic acids, oligonucleotides, genetic therapeutic agents, non-genetic therapeutic agents, vectors for delivery of genetic therapeutic agents, cells, and therapeutic agents identified as candidates for vascular treatment regimens, for example, as agents that reduce or inhibit restenosis. The term "small organic molecule" refers to an organic molecule having 50 or fewer carbon atoms, and fewer than 100 non-hydrogen atoms in total. Generally, exemplary therapeutic agents include, e.g., sirolimus, everolimus, biolimus (e.g., biolimus A9), zotarolimus, tacrolimus and paclitaxel. The therapeutic agent can be amorphous.

In some embodiments, exemplary non-genetic therapeutic agents include anti-thrombogenic agents such as heparin, heparin derivatives, prostaglandin (including micellar prostaglandin E1), urokinase, and PPack (dextrophenylalanine proline arginine chloromethylketone); anti-proliferative agents such as enoxaparin and angiopeptin, monoclonal antibodies capable of blocking smooth muscle cell proliferation, hirudin, and acetylsalicylic acid; anti-inflammatory agents such as dexamethasone, rosiglitazone, prednisolone, corticosterone, budesonide, estrogen, estrodiol, sulfasalazine, acetylsalicylic acid, mycophenolic acid, and mesalamine; anti-neoplastic/anti-proliferative/anti-mitotic agents such as paclitaxel, epothilone, cladribine, 5-fluorouracil, methotrexate, azathioprine, doxorubicin, daunorubicin, cyclosporine, mitomycin, cisplatin, vinblastine, vincristine, epothilones, endostatin, trapidil, halofuginone, and angiostatin; anti-cancer agents such as antisense inhibitors of c-myc oncogene; antimicrobial agents such as triclosan, cephalosporins, aminoglycosides, nitrofurantoin, silver ions, compounds, or salts; biofilm synthesis inhibitors such as non-steroidal anti-inflammatory agents and chelating agents such as thylenediaminetetraacetic acid, O,O'-bis (2-aminoethyl) ethyleneglycol-N,N,N',N'-tetraacetic acid and mixtures thereof; antibiotics such as gentamycin, rifampin, minocyclin, and ciprofloxacin; antibodies including chimeric antibodies and antibody fragments; anesthetic agents such as lidocaine, bupivacaine, and ropivacaine; nitric oxide; nitric oxide (NO) donors such as linsidomine, molsidomine, L-arginine, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warfarin sodium, Dicumarol, aspirin, prostaglandin inhibitors, platelet aggregation inhibitors such as cilostazol and tick antiplatelet factors; vascular cell growth promotors such as growth factors, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; inhibitors of heat shock proteins such as geldanamycin; angiotensin converting enzyme (ACE) inhibitors; beta-blockers; βAR kinase (βARK) inhibitors; phospholamban inhibitors; proteinbound particle drugs such as ABRAXANE™; structural protein (e.g., collagen) cross-link breakers such as alagebrium (ALT-711); and/or any combination and prodrugs of the above.

Exemplary biomolecules include peptides, polypeptides and proteins; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Nucleic acids may be incorporated into delivery systems such as, for example, vectors (including viral vectors), plasmids or liposomes.

Non-limiting examples of proteins include serca-2 protein, monocyte chemoattractant proteins (MCP-1) and bone morphogenic proteins ("BMPs"), such as, for example, BMP-2, BMP-3, BMP-4, BMP-5, BMP-6 (VGR-1), BMP-7 (OP-1), BMP-8, BMP-9, BMP-10, BMP-11, BMP-12, BMP-13, BMP-14, and BMP-15. Preferred BMPs are any of BMP-2, BMP-3, BMP-4, BMP-5, BMP-6, and BMP-7. These BMPs can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNAs encoding them. Non-limiting examples of genes include survival genes that protect against cell death, such as antiapoptotic Bcl-2 family factors and Akt kinase; serca 2 gene; and combinations thereof. Non-limiting examples of angiogenic factors include acidic and basic fibroblast growth factors, vascular endothelial growth factor, epidermal growth factor, transforming growth factors α and β, platelet-derived endothelial growth factor, platelet-derived growth factor, tumor necrosis factor α, hepatocyte growth factor, and insulin-like growth factor. A non-limiting example of a cell cycle inhibitor is a cathespin D (CD) inhibitor. Non-limiting examples of anti-restenosis agents include p15, p16, p18, p19, p21, p27, p53, p57, Rb, nFkB and E2F decoys, thymidine kinase inhibitors and combinations thereof and other agents useful for interfering with cell proliferation.

Exemplary small molecules include hormones, nucleotides, amino acids, sugars, and lipids and compounds having a molecular weight of less than 100kD.

Exemplary cells include stem cells, progenitor cells, endothelial cells, adult cardiomyocytes, and smooth muscle cells. Cells can be of human origin (autologous or allogenic) or from an animal source (xenogenic), or genetically engineered. Non-limiting examples of cells include side population (SP) cells, lineage negative (Lin-) cells including Lin-CD34-, Lin-CD34+, Lin-cKit +, mesenchymal stem cells including mesenchymal stem cells with 5-aza, cord blood cells, cardiac or other tissue-derived stem cells, whole bone marrow, bone marrow mononuclear cells, endothelial progenitor cells, skeletal myoblasts or satellite cells, muscle derived cells, go cells, endothelial cells, adult cardiomyocytes, fibroblasts, smooth muscle cells, adult cardiac fibroblasts + 5-aza, genetically modified cells, tissue engineered grafts, MyoD scar fibroblasts, pacing cells, embryonic stem cell clones, embryonic stem cells, fetal or neonatal cells, immunologically masked cells, and teratoma derived cells. Any of the therapeutic agents may be combined to the extent such combination is biologically compatible.

Examples of medical devices benefiting from use in conjunction with the present disclosure vary widely and can include implantable or insertable medical devices, for example, stents (including coronary vascular stents, peripheral vascular stents, cerebral, urethral, ureteral, biliary, tracheal, gastrointestinal and esophageal stents), stent coverings, stent grafts, vascular grafts, abdominal aortic aneurysm (AAA) devices (e.g., AAA stents, AAA grafts), vascular access ports, dialysis ports, catheters (e.g., urological catheters or vascular catheters such as balloon catheters and various central venous catheters), guide wires, balloons, filters (e.g., vena cava filters, mesh filters, and distal embolic protection devices), embolization devices including cerebral aneurysm filler coils (including Guglielmi detachable coils and metal coils), septal defect closure devices, myocardial plugs, patches, electrical stimulation leads, including leads for pacemakers, leads for implantable cardioverter-defibrillators, leads for spinal cord stimulation systems, leads for deep brain stimulation systems, leads for peripheral nerve stimulation systems, leads for cochlear implants and leads for retinal implants, ventricular assist devices including left ventricular assist hearts and pumps, total artificial hearts, shunts, valves including heart valves and vascular valves, anastomosis clips and rings, tissue bulking devices, and tissue engineering scaffolds for cartilage, bone, skin and other *in vivo* tissue regeneration, sutures, suture anchors, tissue staples and ligating clips at surgical sites, cannulae, metal wire ligatures, urethral slings, hernia "meshes", artificial ligaments, orthopedic prosthesis such as bone grafts, bone plates, fins and fusion devices, joint prostheses, orthopedic fixation devices such as interference screws in the ankle, knee, and hand areas, tacks for ligament attachment and meniscal repair, rods and pins for fracture fixation, screws and plates for craniomaxillofacial repair, dental implants, or other devices that are implanted or inserted into the body and from which therapeutic agent is released.

In some embodiments, suitable medical devices on which a sleeve may be carried include, but are not limited to, those that have a tubular or cylindrical like portion. A tubular portion of a medical device need not be completely cylindrical. The cross-section of the tubular portion can be any shape, such as rectangle, a triangle, etc., not just a circle. Such devices include, but are not limited to, stents, balloons of a balloon catheters, grafts, and valves (e.g., a percutaneous valve). A bifurcated stent is also included among the medical devices which can be fabricated by the methods described herein. The device can be made of any material, e.g., metallic, polymeric, and/or ceramic material.

In some embodiments, examples of balloon materials include relatively non-complaint materials such as polyamides, for instance, polyamide homopolymers and copolymers and composite materials in which a matrix polymer material, such as polyamide, is combined with a fiber network (e.g., Kevlar®: an aramid fiber made by Dupont or Dyneema®, a super-strong polyethylene fiber made by DSM Geleen, the Netherlands). Specific examples of polyamides include nylons, such as nylon 6, nylon 4/6, nylon 6/6, nylon 6/10, nylon 6/12, nylon 11 and nylon 12 and poly(ether-coamide) copolymers, for instance, polyether-polyamide block copolymer such as poly(tetramethylene oxide-b-polyamide-12) block copolymer, available from Elf Atochem as PEBAX. Examples of balloon materials also include relatively complaint materials such as silicone, polyurethane or compliant grades of PEBAX having a larger percentage of poly ether, for example PEBAX 63D. In some embodiments, examples of balloon materials can include semi-compliant polymer materials.

### Method of Manufacture

Where the delivery device is a balloon, the sleeves may be applied to the balloon in a folded state to minimize interactions between the device and the balloon that would have to be disrupted for device delivery, thereby improving release. In some embodiments, sleeves can be made and then applied to a delivery device. For example, a drug delivery sleeve comprising an inner release region, a drug-releasing biodegradable fibrous layer, and an outer adhesive region may be formed and applied to a balloon, which may be folded in certain embodiments.

In some examples, the balloon is manufactured by extrusion technology. Referring to FIG. 9A, biodegradable substrate layer 104 can be formed by spraying or otherwise applying (e.g., dipping, brushing, painting) a polymer/therapeutic agent solution to a cylindrical non-stick template 600 (e.g., a poly(tetrafluoroethylene) "PTFE" template). Once the biodegradable substrate layer is formed, the tissue-adhesive region 106 can be applied to the substrate layer, in a selective manner at predefined patterns, as illustrated in FIG. 9B. Tissue adhesive region 106 can be applied, for example, by dipping, spraying, dropping-on-demand, and/or roll-coating. Upon completion of sleeve formation, the coating apparatus can be dismantled to leave the sleeve 100, which can be removed from the PTFE template, as illustrated in FIG. 9C. The PTFE template can be reused.

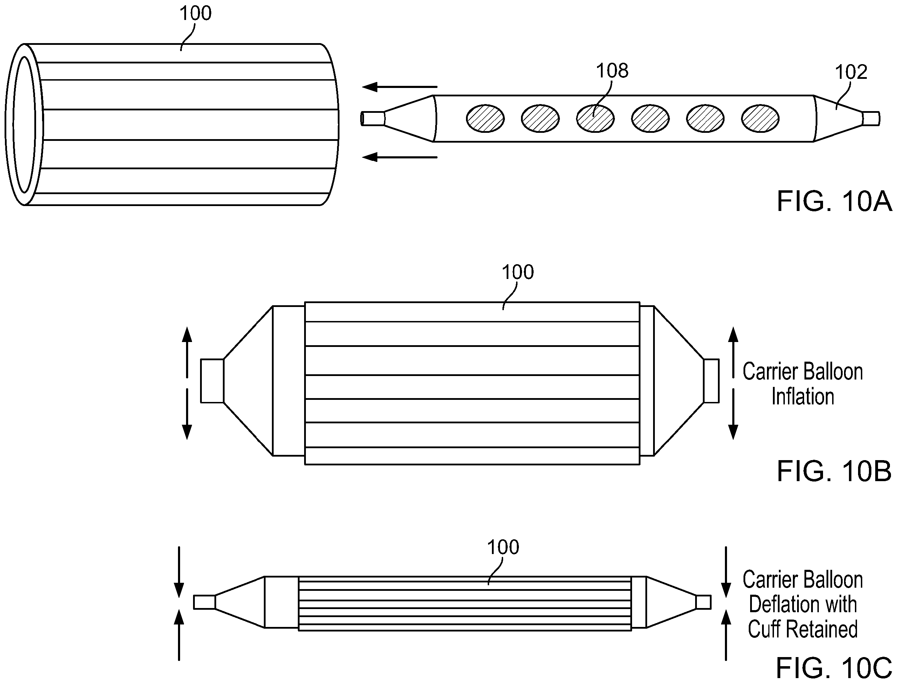

Referring to FIG. 10A, the sleeve 100 can then be introduced over a carrier balloon 102, which can have pre-applied balloon release region 108. Referring to FIG. 10B, the sleeve can be fitted over the device and the carrier balloon can be subsequently inflated to cause a mechanical interaction between the carrier balloon and the sleeve, thereby allowing the release layer to be in contact with the sleeve. Referring to FIG. 10C, deflation can cause the sleeve to follow the balloon to which it is now adhered to and to reform the underlying folded shape of the balloon. To form stacked sleeves, the process is repeated for multiple sleeves with application of inter-sleeve release region (when present) before insertion of a balloon into each overlying sleeve.

In some embodiments, the sleeve includes an elastomeric material so that it can be placed and folded with a balloon carrier, such that when expanded in a treatment site, plastic deformation of the sleeve can be permanently induced. During the balloon folding process, the sleeve is sized to conform to the balloon diameter, ensuring that post balloon folding, a reduced profile is achieved. In some embodiments, no additional balloon-adhesion region is required.

In some examples, rather than an elastomeric material that would wrap around and be folded along with the balloon, a tube of elastomeric material having tissue-adhesive region can be sized such that it can be placed over a folded balloon. In this case, the sleeve and the balloon can have a smaller assembly profile, as the sleeve is not folded together with the balloon. The sleeve can expand with the unfolding balloon during inflation, which can induce plastic deformation of the sleeve, and the sleeve can remain and adhere at the inflated diameter within a treatment site after removal of the balloon catheter.

In some examples, the balloon cones can be puffed (e.g., during packaging and/or device delivery) to ensure securement of the sleeve.

Optionally, a stent may be provided (a) before application of the sleeve (in the event an abluminal sleeve is desired for the stent) or (b) after application of the sleeve (in the event an adluminal sleeve is desired for the stent). As another example, a drug delivery sleeve comprising an inner release region, a first drug-releasing biodegradable substrate layer, a stent, a second drug-releasing biodegradable substrate layer, and an outer adhesive region may be formed and applied to a balloon, which may be folded in certain embodiments. Different drugs may be supplied in the biodegradable substrate layers, for example, an endothelial cell growth promoter may be provided in the inner adlumenal biodegradable substrate layer and an antirestenotic drug may be provided in the outer ablumenal biodegradable substrate layer.

In other examples, sleeves may be formed on the surface of the delivery device. As a specific example (among many other possibilities), a release region may first be applied to a surface of an inflatable balloon. A biodegradable polymer containing layer including a therapeutic agent is then formed over the release region. In a subsequent step, an adhesive region is provided over the biodegradable substrate layer. As a more specific example, a release region may first be applied to a surface of an inflatable balloon formed from a material such as nylon, polyurethane or PEBAX, among others. The release region may comprise, among other possibilities, (a) a shear sensitive adhesive or (b) a zwitterionic release substance such as phosphorylcholine in combination with saline microcapsules (unless a micro-porous or weeping balloon is employed, in which case the saline microcapsules will be excluded). A biodegradable substrate layer, for example, comprising hyaluronic acid and paclitaxel as a therapeutic agent is then formed over the release region, for instance, via a spraying process. The hyaluronic acid in the biodegradable substrate layer may then be crosslinked by applying genipin to the fibrous layer. In a subsequent step, DOPA is applied to the outer fiber layer surface as an adhesive substance, among other possibilities.

Optionally, a stent may be provided (a) before application of the biodegradable substrate layer (in the event an abluminal biodegradable layer is desired), (b) after application of the biodegradable substrate layer (in the event a adluminal biodegradable substrate layer is desired) or (c) after application of one biodegradable substrate layer, followed by formation of another biodegradable substrate layer (in the event that a biodegradable substrate layer-encapsulated stent structure with adluminal and abluminal biodegradable substrate layers is desired).

As noted above, examples of biodegradable substrate layers include non-porous layers (e.g., hydrogel layers) and porous layers (e.g., fibrous layers). Non-porous layers may be provided using techniques such as by dipping, spray coating, coating with an applicator (e.g., by roller, brush, etc), and so forth.

Fibrous layers may be formed using, for example, fiber spinning techniques. For example, electrospinning is a fiber spinning technique by which a suspended drop of polymer (e.g., a polymer in a suitable solvent) is charged with tens of thousands of volts. At a characteristic voltage, the droplet forms a Taylor cone, and a fine jet of polymer releases from the surface in response to the tensile forces generated by interaction of an applied electric field with the electrical charge carried by the jet. This produces a filament of material. This jet can be directed to a grounded surface such as a balloon delivery system and collected as a continuous web of fibers that can be adjusted to give fibers ranging in size, for example, from 50 nm to 100 nm to 250 nm to 500 nm to 1 micron to 2.5 microns to 5 microns to 10 microns to 20 microns. To ensure good coverage, the balloon delivery system may be rotated and reciprocated relative to the jet. Multiple dispensers with differing concentrations of starting materials may be utilized to produce higher concentrations of selected materials in specific areas of the nanofibrous network. Further information on electrospinning may be found, for example, in US 2005/0187605 to Greenhalgh et al. See also Y. Ji et al., "Electrospun three-dimensional hyaluronic acid nanofibrous scaffolds," Biomaterials 27 (2006) 3782-3792.

Porous layers including electrospun fibrous layers increase available surface area and therefore may increase release of any therapeutic agents and increase biodegradation rate relative to nonporous layers. Moreover, such layers may serve to create a scaffold for cell seeding, growth and/or proliferation. For example, in the case of vascular devices, such layers may serve as a scaffold for endothelial cell seeding, growth and/or proliferation *in vivo.*

In some examples, it may be desirable to roughen a surface of interest before performing depositions described herein. For example, a surface may be roughened to provide a series of nooks or invaginations on/within the surface. Any surface may be roughened, e.g., a metallic, polymeric or ceramic surface. Surfaces can be roughened using any technique known in the art. Particularly useful methods for roughening surfaces, such as the surfaces of a stent, are described, e.g., in U.S. Serial No. 12/205,004. The surface of a balloon may also be roughened.

Further, as will be appreciated by skilled practitioners, a biodegradable substrate layer can be deposited on an entire surface of a template or onto only part of a surface of the template. This can be accomplished using template-shielding masks to shield the portions on which coatings are not to be deposited. In some embodiments, the template is a stent. It may be desirable to deposit only on the abluminal surface of the stent. This construction may be accomplished by, e.g. coating the stent before forming the fenestrations. In other embodiments, it may be desirable to deposit only on abluminal and "cutface" surfaces of the stent. This construction may be accomplished by, e.g., depositing on a stent containing a mandrel, which shields the luminal surfaces.

In various embodiments, one or more therapeutic agents may also be included, for example, along with one or more biodegradable polymers in a solution that is used to form a biodegradable substrate layer. As an alternative, a biodegradable polymer and one or more therapeutic agents may be simultaneously deposited (e.g., from separate containers) to form a biodegradable substrate layer. As another alternative, one or more therapeutic agents may be applied (e.g., in solution) to the biodegradable substrate layer after it is formed.

### Examples

### Example 1

A sleeve includes PLGA/Everolimus in a 50:50 composition. The design has a nominal dosage of 1 µg/mm² Everolimus. This sleeve has a tissue adherent layer including a poly(N-isopropylacrylamide) gel matrix and a balloon adherent layer including iopromide. The tissue adhesive region is applied by spraying. The balloon adhesive regions are applied by spraying.

### Example 2

A stacked cuff device includes an inner tubular assembly that includes a sleeve having PLGA/Everolimus in a 50:50 composition. The design has a nominal dosage of 1 µg/mm² Everolimus. An outer tubular assembly includes a sleeve having PLGA/Everolimus in a 50:50 composition. This sleeve has a tissue adherent layer including a poly(N-isopropylacrylamide) gel matrix and a balloon adherent layer including a gelatin-based adhesive. The tissue adhesive region is applied by spraying. The balloon/sleeve adhesive regions are applied by spraying.

### Example 3

A stacked cuff device includes an inner tubular assembly that includes a sleeve having PLA/Paclitaxel in a 90:10 composition. The inner tubular sleeve assembly has a nominal dosage of approximately 1 µg/mm² Paclitaxel. An outer tubular assembly includes a sleeve having PLA/Paclitaxel in a 90:10 composition. The outer tubular sleeve assembly has a nominal dosage of approximately 1 µg/mm² paclitaxel. This sleeve has a tissue adherent layer including a poly(N-isopropylacrylamide) gel matrix and a balloon adherent layer including a gelatin hydrogel glue. The tissue adhesive region is applied by drop-on-demand technology. The balloon/sleeve adhesive regions are applied by drop-on-demand technology.

## Claims

1. A medical device, comprising:
a tubular assembly disposed on an abluminal surface of an expandable balloon (202),
**characterized in that** the tubular assembly comprises a first inner sleeve (300) and a second outer sleeve (200) overlying the first inner sleeve, and a first release region disposed between the first and second sleeves, the sleeves being deliverable to one or more body lumen sites;
the first and second sleeves each comprising:
a substrate layer (204, 304) having an adluminal surface and an abluminal surface, the substrate layer comprising a matrix and a biologically active agent; and
a tissue-adhesive region (206, 306) disposed on the abluminal surface.

2. The medical device of claim 1, wherein the matrix comprises a polymer.

3. The medical device of claim 1, wherein one or both sleeve are biodegradable within a period of about 1 month to about 3 months.

4. The medical device of claim 2, wherein the polymer is selected from the group consisting of polyurethane, polyethylene, polylactic acid, polyglycolic acid, polylactic-co-glycolic acid, poly-DL-lactide, and any combination thereof.

5. The medical device of claim 1, wherein the tissue-adhesive region is configured on the abluminal surface as a plurality of strips, a plurality of dots, a continuous layer, a matrix mesh, a plurality of longitudinal strips, a plurality of circumferential strips, or any combination thereof.

6. The medical device of claim 5, wherein the tissue-adhesive region comprises a repeating pattern of strips, dots, or both.

7. The medical device of claim 1, wherein a second release region (350) is disposed between the first inner sleeve and the abluminal surface of the expandable balloon.

8. The medical device of claim 1, wherein the first release region (250) is adherent to the adluminal surface of the substrate layer of the second outer sleeve.

9. The medical device of claim 7, wherein the second release region is adherent to an abluminal surface of an expandable balloon.

10. The medical device of claim 1, wherein the tissue-adhesive region comprises polyethylene glycol, dextran aldehyde, amino acid-based adhesives, adhesive surface proteins, microbial surface components-recognizing adhesive matrix molecules, fatty ester modified PLA, fatty ester modified PLGA, gel particles, poly(N-isopropylacrylamide) gel particles, or any combination thereof.

11. The medical device of claim 1, wherein the release region comprises contrast agents, proteins, synthetic glues, or any combination thereof.

12. The medical device of claim 1, wherein the biologically active agent is selected from the group consisting of paclitaxel, everolimus, sirolimus, zotarolimus, and biolimus A9, and any combination thereof.

13. The medical device of claim 1, further comprising one or more additional sleeve overlying the second sleeve.

14. The medical device of claim 1, wherein the substrate layers in the first and second sleeves comprise the same or different material.

15. The medical device of claim 1, wherein the medical device comprises a vascular cuff.

## Patentansprüche

1. Medizinprodukt, umfassend:
eine rohrförmige Anordnung, die auf einer abluminalen Oberfläche eines expandierbaren Ballons (202) angeordnet ist,
**dadurch gekennzeichnet, dass** die rohrförmige Anordnung eine erste innere Hülse (300) und eine zweite äußere Hülse (200), die über der ersten inneren Hülse liegt, und eine erste Freisetzungsregion umfasst, die zwischen der ersten und der zweiten Hülse angeordnet ist, wobei die Hülsen an eine oder mehrere Körperlumenstellen abgebbar sind;
wobei die erste und die zweite Hülse jeweils umfassen:
eine Substratschicht (204, 304) mit einer adluminalen Oberfläche und einer abluminalen Oberfläche, wobei die Substratschicht eine Matrix und ein biologisch aktives Mittel umfasst; und
eine Gewebeadhäsionsregion (206, 306), die auf der abluminalen Oberfläche angeordnet ist.

2. Medizinprodukt nach Anspruch 1, wobei die Matrix ein Polymer umfasst.

3. Medizinprodukt nach Anspruch 1, wobei eine oder beide Hülsen innerhalb eines Zeitraums von etwa 1 Monat bis etwa 3 Monaten biologisch abbaubar ist bzw. sind.

4. Medizinprodukt nach Anspruch 2, wobei das Polymer ausgewählt ist aus der Gruppe bestehend aus Polyurethan, Polyethylen, Polymilchsäure, Polyglycolsäure, Poly(milchsäure-co-glycolsäure), Poly-DL-lactid und jeglicher Kombination davon.

5. Medizinprodukt nach Anspruch 1, wobei die Gewebeadhäsionsregion auf der abluminalen Oberfläche als Vielzahl von Streifen, Vielzahl von Punkten, kontinuierliche Schicht, Matrix-Maschenmaterial, Vielzahl längsgerichteter Streifen, Vielzahl umlaufender Streifen oder jeglicher Kombination davon ausgestaltet ist.

6. Medizinprodukt nach Anspruch 5, wobei die Gewebeadhäsionsregion ein sich wiederholendes Muster aus Streifen, Punkten oder beiden umfasst.

7. Medizinprodukt nach Anspruch 1, wobei eine zweite Freisetzungsregion (350) zwischen der ersten inneren Hülse und der abluminalen Oberfläche des expandierbaren Ballons angeordnet ist.

8. Medizinprodukt nach Anspruch 1, wobei die erste Freisetzungsregion (250) an der adluminalen Oberfläche der Substratschicht der zweiten äußeren Hülse haftet.

9. Medizinprodukt nach Anspruch 7, wobei die zweite Freisetzungsregion an einer abluminalen Oberfläche eines expandierbaren Ballons haftet.

10. Medizinprodukt nach Anspruch 1, wobei die Gewebeadhäsionsregion Polyethylenglycol, Dextranaldehyd, Haftstoffe auf Aminosäurebasis, Adhäsionsoberflächenproteine, mikrobielle Oberflächenkomponenten erkennende Adhäsionsmatrixmoleküle (Microbial Surface Components-Recognizing Adhesive Matrix Molecules; MSCRAMMs), Fettester-modifizierte PLA, Fettester-modifizierte PLGA, Gelpartikel, Poly(N-isopropylacrylamid)-Gelpartikel oder jegliche Kombination davon umfasst.

11. Medizinprodukt nach Anspruch 1, wobei die Freisetzungsregion Kontrastmittel, Proteine, synthetische Kleber oder jegliche Kombination davon umfasst.

12. Medizinprodukt nach Anspruch 1, wobei das biologisch aktive Mittel ausgewählt ist aus der Gruppe bestehend aus Paclitaxel, Everolimus, Sirolimus, Zotarolimus und Biolimus A9 und jeglicher Kombination davon.

13. Medizinprodukt nach Anspruch 1, ferner umfassend ein oder mehrere zusätzliche Hülsen, die über der zweiten Hülse liegen.

14. Medizinprodukt nach Anspruch 1, wobei die Substratschichten in der ersten und zweiten Hülse das gleiche Material oder unterschiedliche Materialien umfassen.

15. Medizinprodukt nach Anspruch 1, wobei das Medizinprodukt eine Gefäßmanschette umfasst.

## Revendications

1. Dispositif médical, comprenant :
un ensemble tubulaire disposé sur une surface abluminale d'un ballonnet gonflable (202),
**caractérisé en ce que** l'ensemble tubulaire comprend un premier manchon intérieur (300) et un deuxième manchon extérieur (200) superposé au premier manchon intérieur, et une première région de libération disposée entre le premier et le deuxième manchon, les manchons pouvant être amenés à un ou plusieurs sites de lumières corporelles ;
le premier et le deuxième manchon comprenant chacun :
une couche de substrat (204, 304) ayant une surface adluminale et une surface abluminale, la couche de substrat comprenant une matrice et un agent biologiquement actif ; et
une région d'adhésion aux tissus (206, 306) disposée sur la surface abluminale.

2. Dispositif médical selon la revendication 1, dans lequel la matrice comprend un polymère.

3. Dispositif médical selon la revendication 1, dans lequel un ou les deux manchons sont biodégradables en l'espace d'une période d'environ 1 mois à environ 3 mois.

4. Dispositif médical selon la revendication 2, dans lequel le polymère est choisi parmi le groupe constitué de polyuréthane, de polyéthylène, d'acide polylactique, d'acide polyglycolique, d'acide polylactique-co-glycolique, de poly-DL-lactide, et de toutes combinaisons de ceux-ci.

5. Dispositif médical selon la revendication 1, dans lequel la région d'adhésion aux tissus est configurée sur la surface abluminale sous la forme d'une pluralité de bandelettes, d'une pluralité de points, d'une couche continue, d'un treillis matriciel, d'une pluralité de bandelettes longitudinales, d'une pluralité de bandelettes circonférentielles ou d'une combinaison quelconque de celles-ci.

6. Dispositif médical selon la revendication 5, dans lequel la région d'adhésion aux tissus comprend un motif répétitif de bandelettes, de points, ou les deux.

7. Dispositif médical selon la revendication 1, dans lequel une deuxième région de libération (350) est disposée entre le premier manchon intérieur et la surface abluminale du ballonnet gonflable.

8. Dispositif médical selon la revendication 1, dans lequel la première région de libération (250) adhère à la surface adluminale de la couche de substrat du deuxième manchon extérieur.

9. Dispositif médical selon la revendication 7, dans lequel la deuxième région de libération adhère à une surface abluminale d'un ballonnet gonflable.

10. Dispositif médical selon la revendication 1, dans lequel la région d'adhésion aux tissus comprend du polyéthylène glycol, de l'aldéhyde dextrane, des adhésifs à base d'acides aminés, des protéines de surface adhésives, des molécules matricielles adhésives reconnaissant des composants microbiens superficiels, du PLA modifié par des groupes ester gras, du PLGA modifié par des groupes ester gras, des particules de gel, des particules de gel de poly(N-isopropylacrylamide), ou une combinaison quelconque de ceux-ci.

11. Dispositif médical selon la revendication 1, dans lequel la région de libération comprend des agents de contraste, des protéines, des colles synthétiques, ou une combinaison quelconque de ceux-ci.

12. Dispositif médical selon la revendication 1, dans lequel l'agent biologiquement actif est choisi parmi le groupe constitué de paclitaxel, everolimus, sirolimus, zotarolimus, et biolimus A9, ou une combinaison quelconque de ceux-ci.

13. Dispositif médical selon la revendication 1, comprenant en outre un ou plusieurs manchons supplémentaires superposés au deuxième manchon.

14. Dispositif médical selon la revendication 1, dans lequel les couches de substrat dans le premier et le deuxième manchon comprennent le même matériau ou des matériaux différents.

15. Dispositif médical selon la revendication 1, dans lequel le dispositif médical comprend un manchon vasculaire.
